# FASCICULE DE BREVET EUROPEEN

(11) **EP 3 506 918 B1**
(45) Date de publication et mention de la délivrance du brevet: **19.04.2023**
(21) Numéro de dépôt: 17758863.9
(22) Date de dépôt: 28.08.2017
(51) Int. Cl.: A61K 36/899, A61K 36/15, A61K 38/47, A61P 5/24

(54) **COMPOSITION COMPRENANT DES EXTRAITS DE POLLENS ET/OU DE PISTILS, PROCEDE DE PREPARATION ET UTILISATIONS ASSOCIEES**
ZUSAMMENSETZUNG MIT POLLEN- UND/ODER STEMPELEXTRAKTEN, HERSTELLUNGSVERFAHREN UND ZUGEHÖRIGE VERWENDUNGEN
COMPOSITION COMPRISING POLLEN AND/OR PISTIL EXTRACTS, PREPARATION PROCESS AND ASSOCIATED USES

(30) Priorité: 31.08.2016 FR 1670480
(43) Date de publication de la demande: 10.07.2019
(73) Titulaire: AXEEN PHARMA S.A.R.L., 98000 Monaco (MO)
(72) Inventeur: MORRA, Sossio, 98000 Monaco (MC)
(74) Mandataire: Axe PI
(86) Numéro de dépôt international: PCT/EP2017/071573
(87) Numéro de publication internationale: WO 2018/041790

(56) Documents cités:
- WO-A1-02/17944
- US-A- 5 712 377
- K WINTHER ET AL: "Femal, a herbal remedy made from pollen extracts, reduces hot flushes and improves quality of life in menopausal women: a randomized, placebo-controlled, parallel study", CLIMACTERIC, vol. 8, no. 2, 3 juin 2005 (2005-06-03), pages 162-170, XP055354258, GB ISSN: 1369-7137, DOI: 10.1080/13697130500117987
- PIOTR CZUCZWAR ET AL: "The safety and tolerance of phytotherapies in menopausal medicine - a review of the literature", PRZEGLAD MENOPAUZALNY - MENOPAUSE REVIEW, vol. 1, 1 janvier 2017 (2017-01-01), pages 8-11, XP055419027, PL ISSN: 1643-8876, DOI: 10.5114/pm.2017.67365
- WAGENLEHNER FLORIAN M E ET AL: "A Pollen Extract (Cernilton) in Patients with Inflammatory Chronic Prostatitis-Chronic Pelvic Pain Syndrome: A Multicentre, Randomised, Prospective, Double-Blind, Placebo-Controlled Phase 3 Study", EUROPEAN UROLOGY, vol. 56, no. 3, 3 juin 2009 (2009-06-03), pages 544-551, XP029878678, ISSN: 0302-2838, DOI: 10.1016/J.EURURO.2009.05.046
- ITO R ET AL: "Antiprostatic hypertrophic action of Cernitin pollen-extract (Cernilton(R))", OYO YAKURI - PHARMACOMETRICS, OYO YAKURI KENKYUKAI, JP, vol. 1, 1 janvier 1986 (1986-01-01), pages 1-11, XP009183969, ISSN: 0300-8533
- Hiromi: "Flower Pollen Extract and its Effect on Menopause", , 23 août 2005 (2005-08-23), XP055354257, Extrait de l'Internet: URL:http://www.graminex.com/PDFs/clinicals /Clinicals_Menopause.pdf [extrait le 2017-03-13]
- S. G. IRAÑETA ET AL: "MALDI-TOF MS analysis of labile Lolium perenne major allergens in mixes", CLINICAL & EXPERIMENTAL ALLERGY : JOURNAL OF THE BRITISH SOCIETY FOR ALLERGY AND CLINICAL IMMUNOLOGY, vol. 38, no. 8, 1 août 2008 (2008-08-01), pages 1391-1399, XP055418833, UK ISSN: 0954-7894, DOI: 10.1111/j.1365-2222.2008.03004.x
- Anonymous: "Pollen extraction - an extensive production process? | Allergon Healthcare", , 27 janvier 2015 (2015-01-27), XP055418988, Extrait de l'Internet: URL:https://web.archive.org/web/2015012708 5620/http://www.allergon-healthcare.com/po llen-extraction-extensive-production-proce ss [extrait le 2017-10-25]

## Description

L'invention appartient au domaine technique des formulations comprenant un extrait de pollen et de pistil pour une utilisation diététique ou pharmaceutique ou une utilisation en tant que produit pharmaceutique, supplément diététique ou complément alimentaire, supplément de santé, ou en tant qu'aliment médical/diététique sous forme orale.

Les plantes ont été, et continuent d'être, la première source d'une grande variété de composés médicinaux. Depuis des siècles, des formes variées de produits dérivés de la botanique ont été utilisées pour traiter d'innombrables infections. Les produits botaniques sont généralement retrouvés sous forme de poudres fabriquées à partir d'une ou de plusieurs plantes ou d'extraits dérivés de plantes entières ou de parties de plantes sélectionnées. Ces poudres et extraits sont, pour la plupart, des mélanges complexes de composés biologiques actifs et de composés biologiques inactifs.

Dans les temps modernes, la phytothérapie, bien qu'étant de plus en plus acceptée dans les sociétés Occidentales, fait toujours face à des défis spécifiques. Premièrement, dans l'opinion de beaucoup de médecins hautement qualifiés, la phytothérapie manque de suffisamment de support de données scientifiques dans notre société orientée vers l'hautement technique et scientifique. Deuxièmement, il y a une préoccupation de savoir quels composés d'un remède phytothérapeutique sont efficaces pharmaceutiquement. Par ailleurs, la question se pose concernant les concentrations ou les dosages présents de tels composants pharmaceutiques efficaces des remèdes phytothérapeutiques. En résumé, les médecins traditionnels sont préoccupés par le manque de standards à la fois qualitatifs et quantitatifs des plantes médicinales. Le manque d'une telle standardisation a également amené à une réticence d'une partie des agences règlementaires dans l'acceptation de tels médicaments dérivés de la botanique.

Puisque les traitements à partir de plantes, définis comme des phytothérapies et comme des compositions de plantes biologiquement améliorées, sont dérivés des plantes, la composition chimique de tels traitements à partir de plantes varie selon un nombre de facteurs, notamment la composition génétique, les conditions de pousse dans lesquelles la plante est produite tout comme les conditions de culture et d'isolation des composés actifs de la plante.

Par conséquent, on peut s'attendre à ce que les variants biologiques d'une plante particulière produisent avec des variations significatives en quantités des composés chimiques particuliers présents dans la plante. Pareillement, au sein d'un même variant biologique d'une plante, des différences dans le sol, les moisissures et autres conditions de pousse peuvent significativement affecter la quantité d'un composé chimique spécifique produit par la plante.

Des formulations composées de pollen ou d'extrait de pollen sont connues de l'art antérieur. De telles compositions sont largement utilisées pour leurs influences positives sur la santé, par exemple pour combattre les problèmes causés par la ménopause chez la femme, en tant que stimulant du système immunitaire, en tant qu'inhibiteur des cellules cancéreuses de la prostate, et plus encore. Les pollens (d'abeille) sont actuellement qualifiés comme soi-disant super-aliments ou aliments nutritifs, parce qu'ils sont riches en protéines, sans acides aminés, hydrates de carbone et traces de minéraux.

Les pollens ou les préparations de pollens sont généralement consommés comme un complément alimentaire. Ils peuvent être pris dans leur forme rudimentaire, qui sont les pollens en tant que tels, comme poudre sous forme libre ou encapsulée. Alternativement, les extraits de pollen d'abeille sont également proposés sur le marché. Le document WO2002017944 décrit une formulation composée de deux extraits de pollen séparés. Ces extraits sont obtenus à partir du cytoplasme (partie intérieure de la graine de pollen dépourvue de son enveloppe). Comme l'enveloppe est généralement une source d'allergènes, l'utilisation d'un extrait cytoplasmique a un avantage évident comparé à un pollen naturel.

Il a été montré que des extraits cytoplasmiques purifiés de pollen spécifiques, standardisés, ont une forte teneur en imitateurs de superoxydes dismutases (SOD), flavonoïdes, tanins et polyphénols, tout comme en protéines bénéfiques et en hydrates de carbone. La concentration de composés bénéfiques dans les différents extraits surpasse largement la quantité de composés intéressants dans les pollens à l'état brut.

Les extraits de pollen décrits dans le document WC2002017944 ont chacun leur composition spécifique et sont bénéfiques pour diverses utilisations médicales. Cependant, le contenu de ces extraits a été trouvé comme variant d'un lot à l'autre, et aucune information permettant la standardisation ou le contrôle qualité n'est disponible à ce jour. Ceci n'est pas souhaitable quand un des buts est de vendre une préparation pharmaceutique, un supplément diététique, un complément alimentaire ou de santé ou des aliments médicaux/diététiques.

Les produits actuellement vendus souffrent du fait qu'il y a un manque d'informations convenables qui permettent le contrôle qualité, et la standardisation subséquente des compositions dérivées des pollens.

La présente invention a pour but de fournir un extrait cytoplasmique purifié de pollen spécifique, standardisé, optimisé avec une forte concentration d'agents pour l'amélioration de la santé et de composés bénéfiques, et qui est composé de constituants spécifiques qui permettent la standardisation et le contrôle qualité de la composition comprenant l'extrait. L'extrait est non ou de manière très limitée allergénique ou cytogénique.

La présente invention fournit une composition orale comprenant :
- un extrait de pollen de seigle (*Secale céréale* L.*)* ;
- un extrait de pollen de maïs (*Zea mays L.*) ;
- un extrait de pollen de pin (*Pinus sylvestris L*.) ;
- un extrait de pollen de dactyle (*Dactylis glomerata L.*) ; et
- un extrait de pistil de maïs (*Zea mays L.*).

Elle se caractérise en ce que lesdits extraits comprennent une protéine ou un peptide dérivé de ladite protéine qui est de la bêta-1,3-glucanase, ladite composition étant préférentiellement sous la forme d'un comprimé, une gélule, un gel mou, un semi-solide, un solide, un liquide ou une poudre.

Elle a également pour objet un procédé de préparation d'un extrait aqueux de pollen et de pistil de plante(s) appartenant préférentiellement à la famille des pinacées et/ou des graminées (poacées) comprenant les étapes successives de :
a) extraction aqueuse de pollen ;
b) extraction aqueuse de pollen et de pistil ;
c) séchage par atomisation des extraits obtenus aux étapes a) et b) ;
d) récupération desdits extraits de pollen et de pistil de plante(s) obtenus en c) ;
caractérisé en ce que la température des extractions est strictement inférieure à 45°C.

Elle a encore pour objet un extrait aqueux de pollen, ou de cytoplasme de pollen, et de pistil susceptible d'être obtenu selon le procédé selon l'invention.

Enfin, elle a pour objet une composition ou un extrait selon l'invention, pour son utilisation :
- dans le traitement des symptômes de la péri-ménopause, post-ménopause, ménopause, de préférence la ménopause chez la femme ;
- dans le traitement du syndrome prémenstruel (SPM), préférentiellement dans le traitement des troubles dysphoriques prémenstruels ;
- dans le traitement des bouffées de chaleurs induites par l'hormonothérapie chez les patients présentant un cancer ; et
- dans le traitement des symptômes de l'andropause et d'inconforts relatifs aux déséquilibres hormonaux associés chez l'homme.

L'invention est exposée dans le jeu de revendications joint.

La composition est particulièrement avantageuse en ce qu'elle fournit une large concentration d'agents promouvant la santé grâce à l'utilisation de différents matières végétales et d'espèces de plantes. De plus, la composition est fortement reproductible entre différents lots (en termes de qualité et d'ingrédients présents), ce qui permet un contrôle qualité, une standardisation, une traçabilité des lots et un profilage de protéines reproductibles. Elle est ainsi en conformité avec les bonnes pratiques de fabrication des produits pharmaceutiques et des compléments alimentaires sur le marché (produits botaniques de qualité pharmaceutique). Cette reproductibilité donne également la garantie que chaque nouveau lot d'extrait de pollen satisfait aux spécifications établies et de ce fait à la même activité physiologique que les lots utilisés pour les études cliniques. Le procédé de fabrication permet d'obtenir le composé bénéfique recherché.

Le manque de niveaux standards des composés pharmaceutiquement actifs dans les produits botaniques naturels a abouti à une réticence des prestataires de soin de santé à prescrire ces produits à leurs patients.

La présente invention concerne des compositions orales qui comprennent des extraits de pollen de plante et des pistils de plante et dans lesquels la présence d'un ou plusieurs marqueurs de protéines et/ou peptide dérivé des protéines, dont une protéine qui est de la bêta-1,3-glucanase ou un peptide dérivé de ladite protéine, est détectable,. Ces marqueurs de protéines sont une bonne indication de la qualité de la composition finale. Ces marqueurs peuvent être également utilisés pour la standardisation des compositions.

Selon l'invention, on parle d'extraction lorsqu'on utilise un solvant, avantageusement respectueux de l'environnement comme par exemple l'eau, la glycérine, les glycols, les éthers, les huiles, les mélanges hydroalcooliques, l'éthanol et les autres alcools, sur une matière première végétale pour en extraire certains composés ou molécules, après un éventuel mélange, décantation et filtration.

Le solvant peut être ensuite éliminé partiellement ou totalement pour obtenir un extrait.

Après leur ramassage, les pollens peuvent être utilisés frais ou séchés, avantageusement séchés, et éventuellement débactérisés.

Les extraits de pollen et de pistil selon l'invention peuvent être des extraits huileux et/ou aqueux.

Par extrait huileux, on entend un extrait contenant des principes actifs liposolubles obtenu par extraction, par exemple une macération, une infusion, une digestion, une décoction, une percolation ou encore une lixiviation, préférentiellement une macération à température ambiante comprise entre 15 et 27°C, d'une matière première végétale dans un solvant huileux tel qu'un éther, une cétone ou une huile.

A titre d'exemples non limitatifs, le solvant huileux est un éther tel que l'éther diéthylique ou une cétone telle que l'acétone.

Par extrait aqueux, on entend un extrait contenant des principes actifs hydrosolubles obtenu par extraction, par exemple une hydrodistillation, une macération, une infusion, une digestion, une décoction, une percolation ou encore une lixiviation, préférentiellement une macération à basse température comprise entre 15 et 40°C, d'une matière première végétale dans un solvant aqueux, c'est-à-dire un solvant comprenant de l'eau prise seule ou avantageusement en mélange avec d'autre(s) solvant(s) tels qu'un alcool, une cétone, et/ou un tensioactif non ionique.

A titre d'exemples non limitatifs, le solvant aqueux est choisi parmi un mélange comprenant majoritairement de l'eau en combinaison avec un alcool tel que l'éthanol, une cétone telle que l'acétone, et/ou tensioactif non ionique.

La composition utilisée selon l'invention comprend préférentiellement un extrait aqueux de pollen et/ou de pistil, plus préférentiellement un extrait aqueux de pollen et de pistil.

Selon l'invention, la composition orale comprend plusieurs extraits provenant d'une matière végétale choisie parmi pollen et des pistils.

Les pollens qui sont utilisés dans la présente composition proviennent de plantes appartenant aux familles des graminées (poacées) et des pinacées.

Les poacées (*Poaceae),* appelées également graminées *(Gramineae),* sont une famille de plantes monocotylédones de l'ordre des Poales. Cette famille, composée d'environ 12 000 espèces regroupées en 780 genres, comprend la plupart des espèces appelées communément « herbes » et « céréales ». Ce sont généralement des plantes herbacées, plus rarement ligneuses (bambous).

Comme tous les pollens anémophiles, le pollen des poacées est de forme sphérique ou légèrement ellipsoïdale à ornementations réduites. L'aperture unique (ou pore) est ronde : c'est un des critères des Monocotylédones. Le pollen des poacées est de petite taille et léger. La taille est de l'ordre de 40 microns. Pour les céréales, la taille est de 60 à 100 microns.

La famille des pinacées (*Pinaceae*), ou abiétacées, regroupe des plantes gymnospermes ; elle compte 220-250 espèces réparties en 11 genres. Ce sont des arbres ou des arbustes, des régions tempérées, soit à feuilles persistantes en aiguille ou en écailles, soit caduques comme celles des mélèzes. Dans cette famille, les espèces indigènes en France se trouvent parmi les genres *Abies* (les sapins), *Picea* (les épicéas), *Larix* (le mélèze d'Europe), *Pinus* (les pins).

Les pinacées produisent de gros grains de pollen de manière abondante dont la taille est généralement comprise entre 40 et 100 microns. Ils sont dépourvus de pores. Les grains de pollen des pins, sapins, épicéa et cèdre possèdent deux ballonnets qui facilitent leur suspension dans l'air. Les grains de pollen des mélèzes et des douglas sont plus ou moins sphériques et sans ballonnet.

Les plantes à partir desquelles les pollen et pistil sont obtenus sont choisies parmi les genres *Secale, Zea, Pinus* et/ou *Dactylis*, ou un mélange de ces derniers.

Plus particulièrement, tel que cela est défini à la revendication 1, les plantes à partir desquelles les pollen et/ou pistil sont obtenus sont choisies parmi les espèces *Secale céréale L.* (seigle), *Zea mays L.* (maïs), *Pinus sylvestris L.* (pin), et/ou *Dactylis glomerata L.* (dactyle), ou un mélange de ces derniers.

De façon avantageuse, les extraits de pollen sont obtenus à partir du cytoplasme (partie intérieure de la graine de pollen dépourvue de son enveloppe). Comme l'enveloppe est généralement une source d'allergènes, et un obstacle à la disponibilité des composés du cytoplasme, l'utilisation d'un extrait cytoplasmique de pollen présente un avantage évident comparé à l'utilisation d'un extrait de pollen naturel. De tels extraits cytoplasmiques purifiés de pollen spécifiques, standardisés, ont une forte teneur en imitateurs de superoxydes dismutases (SOD), flavonoïdes, tanins et polyphénols, tout comme en protéines bénéfiques et en hydrates de carbone. La concentration de composés bénéfiques dans les différents extraits est largement supérieure à la quantité de composés intéressants dans les pollens à l'état brut.

Ainsi, bien que les plantes des deux familles soient connues pour entraîner des réactions allergiques, les extraits de la présente invention ne sont pas, ou de manière très limitée allergéniques comme ils ne comprennent pas ou seulement des traces d'allergènes. Les extraits de pollen et de pistil de plantes provenant des graminées et des pinacées ont été montrés comme comprenant une abondance de composants et de molécules promouvant la santé, tel que les SODs. Dès lors, leur utilisation peut avoir de multiples effets bénéfiques sur les inconforts, les maladies et la santé humaine en général.

Le terme « composant d'une composition » doit être entendu comme un constituant de la composition qui est composé d'un ou plusieurs ingrédients actifs.

Ainsi, plus particulièrement, un composant dérivé d'un ou plusieurs extraits est équivalent à un ou plusieurs extraits perse.

L'expression « % en poids » ou « w/w » (pourcentage en poids), ici et tout au long de la description sauf s'il en est précisé autrement, se réfère au poids relatif des composés respectifs par rapport au poids total de la formulation.

Le terme « excipient » doit être entendu comme une substance naturelle ou synthétique formulée pour être utilisée avec un ingrédient actif inclus dans le mélange dans le but de regrouper les mélanges contenant des ingrédients actifs ou pour conférer une amélioration thérapeutique à l'ingrédient actif dans la forme de dosage finale, telle que pour faciliter l'absorption du médicament ou sa solubilité, ou pour aider à la fabrication du mélange.

Dans cette description, à moins qu'il ne soit spécifié autrement, il est entendu que, lorsqu'un intervalle est donné, il inclut les bornes supérieure et inférieure dudit intervalle.

La composition selon l'invention se caractérise en ce que les extraits comprennent au moins une protéine ou un peptide dérivé de ladite protéine qui est de la bêta-1,3-glucanase.

Selon l'invention, la présence de marqueurs spécifiques de protéines ou de peptides provenant de ces protéines (marqueurs) peut être confirmée dans la composition finale, lesdits marqueurs étant révélateurs de la qualité de la composition et peuvent donc être utilisés pour la standardisation et le contrôle qualité.

En effet, le Demandeur a pu mettre en évidence que la présence de certains traceurs ou marqueurs (protéine ou peptide dérivé de ladite protéine) permettaient de confirmer la présence de certains extraits de pollen et/ou de pistil dans la composition finale.

Ainsi, le Demandeur a pu démontrer que les protéines suivantes ou peptides dérivés desdites protéines permettent la traçabilité des pollens et/ou pistils suivants :
- l'allergène de pollen Sec 4 (*Secale cereale*) (Q5TIW8 et Q5TIW7), la glucanendo- 1,3-beta-D-glucosidase (Q1 EM97) permettent par exemple de tracer la présence du pollen *Secale cereale L.* ;
- l'oxydase de réticuline (Reticulase Oxidase) (B6T5D7), la beta-1,3-glucanase (E1AFV5), la pectinesterase (B6UCK8), la protéine Extensin-like (Q9SPM0), l'exopolygalacturonase (PGLR2), la beta-amylase (Q9SYS1), la chitinase (D0EM57) permettent par exemple de tracer les pistils et pollens de *Zea mays L. ;*
- l'allergène de pollen Lol p 4 permet par exemple de tracer la présence du pollen *Dactylis glomerata L.* ; et
- la protéine de transfert lipidique permet par exemple de tracer la présence du pollen *Pinus sylvestris L. et*/*ou Zea mays L..*

Lesdites protéines ou lesdits peptides dérivés desdites protéines présents dans les extraits finaux et de fait dans la composition selon l'invention sont au moins la bêta-1,3-glucanase.

Les extraits de la composition objet de l'invention comprennent avantageusement au moins une deuxième protéine ou un peptide dérivé de ladite deuxième protéine choisis parmi le groupe de l'allergène de pollen Lol p 4 de Dactylis *glomerata L.,* de l'allergène de pollen Sec 4 (*Secale cereale*), la glucanendo-1,3-beta-D-glucosidase, la beta-amylase, la chitinase ou toute combinaison de ces dernières.

Le Demandeur a pu mettre en évidence que l'absence d'un ou plusieurs de ces marqueurs ou traceurs, spécifiques de certains pollens et/ou pistils, dans les extraits contenant a *priori* lesdits pollens et/ou de pistils et de fait dans les compositions comprenant a *priori* de tels extraits était des indicateurs qualitatifs des produits.

Ainsi, l'absence d'un marqueur spécifique d'un pollen et/ou d'un pistil donné indique que l'extrait ou la composition ne comprend pas ledit pollen et/ou pistil, ou qu'ils ont été dénaturés, rendant ainsi la composition comprenant au moins un tel extrait de pollen et/ou de pistil dégradé, inefficace ou, à tout le moins, moins efficace.

La production d'une composition orale à partir d'extraits de plantes requiert souvent des étapes de production spécifiques tel que le séchage par atomisation, la granulation, etc. qui peuvent avoir un impact sur la composition finale et ses constituants telles que les protéines. Certaines protéines qui sont présentes dans la matière végétale brute sont susceptibles, à cause de la nature spécifique du procédé de fabrication (typiquement le séchage par atomisation), de perdre leur activité ou d'être perdues ou dégradées. Les inventeurs de la présente invention ont trouvé que l'utilisation d'un procédé de séchage par atomisation optimisé (différence de température de 50°C entre la température d'entrée et celle de sortie) évitait la dénaturation de protéines cibles concernées. Les inventeurs de la présente invention ont également trouvé que la présence dans la composition finale d'au moins une de ces protéines ou peptides dérivés de ces protéines depuis les protéines listées assure que la qualité de la composition et des extraits utilisés dans la composition sont optimaux et de qualité pharmaceutique et que l'activité de la composition n'est pas altérée. Par ailleurs, en fournissant un contrôle qualité adéquat, une meilleure uniformité du contenu peut être assurée, ce qui est important quand on traite des patients pour des symptômes spécifiques et/ou des inconforts.

De façon avantageuse, la composition selon l'invention comprend :
- un extrait aqueux de pollen de *Secale céréale L.* ;
- un extrait aqueux de pollen de *Zea mays L.* ;
- un extrait aqueux de pollen de *Pinus sylvestris L.* ;
- un extrait aqueux de pollen de *Dactylis glomerata L.* ; et
- un extrait aqueux de pistil de *Zea mays L..*

Préférentiellement, la composition selon l'invention comprend :
- 45%à 90% d'extrait aqueux de pollen de *Secale céréale L.* en poids du poids total de l'extrait ;
- 1% à 35% d'extrait aqueux de pollen de *Zea mays L.* en poids du poids total de l'extrait ;
- 0,01% à 5% d'extrait aqueux de pollen de *Pinus sylvestris L.* en poids du poids total de l'extrait ;
- 3% à 30% d'extrait aqueux de pollen de *Dactylis glomerata L.* en poids du poids total de l'extrait ; et
- 0,1% à 10% d'extrait aqueux de pistil de *Zea mays L.* en poids du poids total de l'extrait.

De préférence, la dose journalière des extraits aqueux de pollen et/ou de pistil de la composition selon la présente invention est comprise entre 160 mg et 480 mg, plus préférentiellement entre 160 et 320 mg. Cette dose journalière est préférentiellement administrée en 1, 2 ou 3 prises (matin, midi et/ou soir) par exemple sous forme de 1, 2, 3, 4 ou 6 comprimés.

Ainsi, compte tenu des extraits de pollens et/ou de pistils préférentiellement compris dans la composition selon l'invention, ladite composition comprend au moins deux marqueurs ou traceurs qui sont des protéines ou peptides dérivés du groupe tel que décrit ci-dessus, de préférence encore au moins trois, plus préférablement au moins quatre.

Selon la présente invention, la protéine ou le peptide dérivé qui est la bêta-1,3-glucanase, est identifiable dans la composition selon la présente invention.

Plus préférentiellement encore, la composition objet de l'invention comprend au moins deux marqueurs dont :
- le premier marqueur est la beta-1,3-glucanase ;
- le deuxième marqueur est l'allergène de pollen Sec 4 (*Secale cereale*).

En effet, de façon surprenante, le Demandeur a pu mettre en évidence que les marqueurs ci-dessus étaient particulièrement sensibles et n'étaient pas présents dans les compositions de l'art antérieur, qui ne sont pas préparées selon un procédé reproductible tel que le procédé objet de l'invention.

Ainsi, selon un premier mode de réalisation de l'invention, la composition objet de l'invention comprend d'une part :
- un extrait de pollen de *Secale cereale L.* ;
- un extrait de pollen de *Zea mays L.* ;
- un extrait de pollen de *Pinus sylvestris L.* ;
- un extrait de pollen de *Dactylis glomerata L.* ; et
- un extrait de pistil de *Zea mays* L ;
et d'autre part au moins les marqueurs suivants :
- bêta-1,3-glucanase (pour tracer la présence du pollen de *Zea mays L.*) ; et
- l'allergène de pollen Sec 4 (pour tracer la présence des pollens et pistils de *Secale céréale*).

Selon un deuxième mode de réalisation de l'invention, la composition objet de l'invention comprend d'une part :
- un extrait de pollen de *Secale céréale L.* ;
- un extrait de pollen de *Zea mays L.* ;
- un extrait de pollen de *Pinus sylvestris L.* ;
- un extrait de pollen de *Dactylis glomerata L.* ; et
- un extrait de pistil de *Zea mays* L ;
et d'autre part au moins les marqueurs suivants :
- l'allergène de pollen Sec 4 (*Secale cereale*) (Q5TIW8 et Q5TIW7) et/ou la Glucanendo-1,3-beta-D-glucosidase (Q1EM97) (pour tracer la présence du pollen *Secale céréale L.)* ;
- l'oxydase de réticuline (Reticulase Oxidase) (B6T5D7), la Beta-1,3-glucanase (E1AFV5), la Pectinesterase (B6UCK8), la protéine Extensin-like (Q9SPMO), l'Exopolygalacturonase (PGLR2), la Beta-amylase (Q9SYS1) et/ou la chitinase (D0EM57) (pour tracer les pistils et pollens de *Zea mays L.*) ;
- l'allergène de pollen Lol p 4 (pour tracer la présence du pollen *Dactylis glomerata L.)* ; et
- la protéine de transfert lipidique (qui permet par exemple de tracer la présence du pollen *Pinus sylvestris L. et*/*ou Zea mays L*.).

De façon particulièrement avantageuse, la composition utilisée selon l'invention comprend un marqueur ou traceur pour chacun des pollen et/ou pistil extrait.

L'invention a également pour objet un procédé de préparation d'un extrait de pollen et de pistil de plante(s) appartenant préférentiellement à la famille des pinacées et/ou des poacées comprenant les étapes successives de :
a) extraction aqueuse de pollen ;
b) extraction aqueuse de pollen et de pistil ;
c) séchage par atomisation des extraits obtenus aux étapes a) et b) ;
d) récupération desdits extraits de pollen et de pistil de plante(s) obtenus en c) ;
e) dont la température des extractions est strictement inférieure à 45°C.

Le Demandeur a pu mettre en évidence que l'étape d'extraction était particulièrement sensible. Ainsi, la température de l'extraction doit être strictement inférieure à 45°C. Au-delà de cette température, un ou plusieurs marqueurs des pollens et pistils détaillés ci-dessus ne sont plus présents. La qualité de la composition et/ou son efficacité ne sont donc pas assurées.

Préférentiellement, la température de l'extraction doit être inférieure à 42°C.

La durée de l'étape d'extraction, pour chacun des extraits, est préférentiellement d'au moins 6h, préférentiellement au moins 10h, plus préférentiellement encore au moins 12h.

De plus, le Demandeur a pu mettre en évidence qu'une séparation dans des conditions trop soutenues dégradait le ou les extraits. Ainsi, lorsque l'extraction est couplée à une séparation, celle-ci ne doit pas dépasser les 6000 tours par minute (tr/min), préférentiellement 4500 tr/min, plus préférentiellement encore 2800 tr/min.

Le procédé ainsi développé permet de garantir une bonne traçabilité des pollens et pistils utilisés. En effet, le Demandeur a pu mettre en évidence que les extraits fabriqués selon les procédés de l'art antérieur ne permettent pas de retrouver la présence d'un ou plusieurs marqueurs décrits ci-dessus et donc, de facto, de la présence de l'ensemble des extraits de pollens et/ou de pistils dans la composition.

Les compositions de l'art antérieur ne sont donc pas homogènes et ne contiennent pas, dans la composition finale, l'ensemble des extraits de pollens et/ou de pistils attendus.

Le procédé objet de l'invention comprend avantageusement des étapes additionnelles de séparation, de filtration et/ou d'évaporation permettant d'augmenter la concentration de l'extrait final ou d'optimiser la préparation dudit extrait.

Le procédé objet de l'invention présente par ailleurs les avantages suivants :
- il permet une standardisation ;
- il présente une excellente reproductibilité ;
- il permet une réduction des coûts en optimisant et limitant le nombre d'opérations de l'outil industriel.

Il permet également de pouvoir tracer la présence des extraits dans la composition selon l'invention et ainsi assurer sa qualité et par là même son efficacité.

Parmi les avantages permettant de réduire les manipulations et les coûts, on peut citer :
- une planification simplifiée ;
- une occupation des équipements optimisés ;
- des temps improductifs réduits (nettoyage, changement de lot, nombre de validations, *etc*.) ;
- une gestion du nombre de référence limitée ;
- une diminution des opérations à risque (nettoyage, pesées, procédures de travail, coûts réduits en validation, stabilité, formulation du comprimé simplifiée, risque microbiologique, traçabilité, *etc.*).

Compte tenu du fait que le procédé de préparation selon l'invention permet d'obtenir un extrait de pollen et de pistil contenant des marqueurs non indentifiables lors de l'utilisation de procédé de préparation d'extrait selon l'art antérieur, l'invention a également pour objet l'obtention d'un extrait aqueux de pollen, ou de cytoplasme de pollen, et de pistil appartenant à la famille des pinacées et/ou des poacées susceptible d'être obtenu selon le procédé de préparation décrit ci-dessus.

De préférence, elle a pour objet un extrait aqueux de pollen et/ou de pistil de *Secale céréale L.,* de *Zea mays L.,* de *Pinus sylvestris L.* et/ou de *Dactylis glomerata L.,* susceptible d'être obtenu selon le procédé de préparation comprenant les étapes suivantes de :
a) extraction à l'eau de pollens de *Secale céréale L.,* de *Zea mays L.,* de *Pinus sylvestris L.* et/ou de *Dactylis glomerata L.,* à une température inférieure à 45°C afin d'obtenir un premier extrait ;
b) extraction à l'eau de pollens et de pistils de *Zea mays L.,* à une température inférieure à 45°C afin d'obtenir un second extrait ;
c) mélange du premier et du second extraits obtenus en a) et b) ;
d) séchage par atomisation du mélange obtenu en c) ;
e) récupération du mélange d'extraits de pollen et de pistil obtenu en d).

Préférentiellement, la température de l'extraction, pour chacun des extraits, doit être inférieure à 42°C.

La présence des protéines susmentionnées dans la composition finale peut être confirmée au moyen de n'importe laquelle des méthodes appropriées connues de l'art qui permettent une identification. Selon une variante préférée, ladite analyse est effectuée via des moyens de spectrométrie de masse (SM). Selon une variante plus préférée, ladite méthode d'analyse est une chromatographie en phase liquide (CPL) ou une CLHP combinée avec une spectrométrie de masse (SM). Des techniques appropriées sont CPL-SM avec une carte peptidique massique ou un tandem SM (CPL-SM/SM). De préférence, lesdites protéines et/ou lesdits peptides dérivés de protéines sont identifiables par chromatographie en phase liquide - spectrométrie de masse.

Selon une autre variante, les extraits proviennent de matières végétales tels que les pollens et/ou les pistils, dans laquelle les plantes appropriées sont sélectionnées à partir du groupe suivant : maïs (*Zea mays L*.), seigle (*Secale céréale L.),* dactyle (*Dactylis glomerata L.*) et pin (*Pinus sylvestris L*.)*,* ou toute combinaison de ce qui précède. Il a été trouvé par les inventeurs que ces espèces de plantes fournissent des extraits de qualité, qui sont très bénéfiques pour leur utilisateur quand ils sont consommés oralement.

Avantageusement, les protéines et/ou peptides marqueurs susmentionnés qui sont utilisés pour vérifier la qualité du produit peuvent être dérivés depuis une espèce de plante spécifique. Par exemple, on peut vérifier si oui ou non la qualité du matériel utilisé d'une espèce de plante spécifique utilisée dans la composition était de qualité optimale, ou si une des origines était mauvaise. Ceci permet une traçabilité durant la production entière de la composition, depuis la récolte de la plante jusqu'à la composition finale.

Selon une autre ou une variante supplémentaire, lesdites oxydase de réticuline, endochitinase A, bêta-1,3-glucanase, exopolygalacturonase proviennent du maïs (*Zea mays L.*).

Selon une autre ou une variante supplémentaire, ladite protéine de transfert lipidique non spécifique provient du pin (*Pinus sylvestris L. et*/*ou Zea mays L*.).

Comme indiqué précédemment, selon une autre variante préférée de l'invention, lesdits extraits proviennent de matières végétales provenant de *Zea mays L., Secale céréale L., Dactylis glomerata L., Pinus sylvestris* L. ou un mélange de ces derniers.

De préférence, la matière végétale utilisée pour les extraits est fraichement récoltée. Les pollens utilisés pour la présente invention peuvent être des pollens récoltés par des insectes (tel que du pollen d'abeille) ou récoltés par une intervention humaine. Le pollen d'abeille par exemple contient du pollen, mais aussi du nectar et de la salive d'abeille. Le pollen récolté via une intervention humaine est dépourvu de tels ingrédients additionnels. De préférence, lesdits pollens des présentes compositions sont obtenus seulement par une intervention humaine. Ceci permet une fois de plus une standardisation du produit final.

Les compositions selon la présente invention sont riches en imitateurs de SOD, flavonoïdes, tannins, polyphénols, vitamines, enzymes et oligoéléments et ont été montrées pour avoir de nombreux effets sur la santé, concernant spécialement les effets sur la santé de la femme. Les compositions selon la présente invention ne contiennent pas d'hormones, tels que les phytoestrogènes.

La composition selon la présente invention est également riche en acides aminés. De préférence, la quantité totale des acides aminés de la composition est d'au moins 20 g/kg, plus préférablement d'au moins 25 g/kg, tel que par exemple 26,3 g/kg. Cette dernière peut également servir comme contrôle qualité additionnel pour la présente composition mais est aussi avantageux pour les utilisateurs, en tant que source d'acides aminés essentiels.

A titre illustratif ne relevant pas de l'invention, la demande est également dirigée vers une méthode de production de plusieurs extraits comprenant plusieurs pollens de cytoplasme purifiés spécifiques, standardisés, provenant d'une matière végétale choisie parmi du pollen et des pistils. Ladite méthode comprend la récolte de la matière végétale, *i.e*. le pollen et/ou les pistils de fleurs. La récolte a lieu préférentiellement seulement par une intervention humaine, dans le but de limiter les variations dans les extraits finaux et une influence externe.

La matière végétale provient des plantes appartenant aux familles des graminées et/ou des pinacées. Plus particulièrement, ladite matière végétale provient de plantes sélectionnées parmi le groupe du maïs (e.g. *Zea mays L*.), du seigle (e.g. *Secale céréale L.*), du dactyle (e.g. *Dactylis glomerata L*.) et du pin (e.g. *Pinus sylvestris* L.). Ladite matière végétale est au moins dérivée d'un mélange de pollens de maïs (*Zea mays L.*) et de seigle. Pour d'autres compositions préférées, nous nous référons à la description au-dessus, qui est à comprendre comme étant entièrement comprise dans cette section également. La fraction cytoplasmique est extraite ultérieurement depuis la fraction solide par addition de solvant tel que l'acétone en milieu aqueux. Les fractions solides et liquides sont ultérieurement séparées, et la fraction solide peut être soumise à une deuxième extraction si cela est jugé souhaitable. Les extraits cytoplasmiques de pollen purifiés obtenus, spécifiques, standardisés, peuvent être mixés (différents extraits provenant de différents mélanges de matières végétale peuvent y être rajoutés en même temps). Dans une étape ultérieure, les extraits sont séchés pour obtenir une poudre, par exemple par séchage par atomisation.

La poudre obtenue est ensuite utilisée pour la fabrication d'un comprimé, avec un ou plusieurs extraits, dans lequel lesdits extraits sont des extraits séchés tels que décrits au-dessus.

Les inventeurs ont trouvé que les bénéfices sur la santé associés à la composition selon la présente invention, avec des extraits de pollens cytoplasmiques purifiés spécifiques, standardisés, d'un mélange de matières végétales de plusieurs plantes sont plus importants en comparaison avec des compositions qui comprennent des extraits d'une seule plante seulement.

La composition objet de la présente invention peut avantageusement contenir en outre un composé dérivé de plantes choisies parmi *Hypericum sp.*, telle que *Hypericum perforatum*, *Crocus sp*., telle que *Crocus sativus, Lavendula sp*. (lavande), *Rhodiola sp*. telle que *Rhodiola rosea, Echium sp*. telle que *Echium amoenum, Verbena sp*. telle que *Verbena officialis, Avena sp*. telle que *Verbena sativa, Eleutherococcus sp*. telle que *Eleutherococcus senticosus, Leonurus sp*. telle que *Leonurus cardia* et *Schisandra sp*. telle que *Schisandra chinensis.*

De façon avantageuse, le composé actif est du safranal et/ou de la picro(crocine).

La concentration en safranal et/ou en picro(crocine) est avantageusement supérieure à 0,001% en poids du poids total de la composition, de préférence comprise entre 0,001 % et 10%, de préférence entre 0,01% et 1%.

Le safranal et/ou la (picro)crocine se trouvent généralement dans le safran. Ces composés sont connus pour être utiles dans le traitement et/ou la prévention de la dépression de la dysphorie ou des symptômes liés à ces troubles.

De façon surprenante, le Demandeur a pu mettre en évidence qu'en ajoutant du safranal et/ou de la (picro)crocine à la composition selon l'invention, on améliore le traitement des femmes souffrant du syndrome prémenstruel (SPM ou PMS pour "premenstrual syndrome" en anglais).

Le syndrome prémenstruel (SPM) est un ensemble de symptômes physiques et émotionnels qui surviennent habituellement de 2 à 7 jours avant les règles (parfois jusqu'à 14 jours). Ils prennent généralement fin avec l'arrivée des règles ou dans les quelques jours qui les suivent.

Les symptômes les plus courants sont une fatigue prononcée, les seins sensibles et gonflés, un gonflement du bas-ventre, des maux de tête et de l'irritabilité. Près de 75 % des femmes fécondes éprouvent des symptômes légers à la veille ou au moment de leurs règles, comme des crampes légères de l'utérus.

Ainsi, une telle composition est particulièrement efficace et permet un apport de tonus et de vitalité, permet une réduction de la fatigue, aide à maintenir une humeur positive, favorise le confort avant et pendant les règles.

20 % à 30 % des femmes ont des symptômes suffisamment intenses pour interférer avec leurs activités quotidiennes. Parmi les syndromes, le trouble dysphorique prémenstruel (TDPM) est une forme sévère du syndrome prémenstruel (SPM) avec au premier plan des symptômes psychiatriques, survenant durant la dernière semaine de la phase lutéale et s'améliorant au début de la phase folliculaire. Les caractéristiques essentielles du TDPM sont : une humeur dépressive, une anxiété et une labilité émotionnelle marquées, ainsi qu'une diminution de l'intérêt pour les activités de la vie quotidienne. Il toucherait de 2 % à 6 % des femmes.

Ainsi, la composition objet de l'invention est avantageusement utile et efficace pour son utilisation dans le traitement du syndrome prémenstruel (SPM) et plus particulièrement pour son utilisation dans le traitement des troubles dysphoriques prémenstruels.

La composition selon l'invention est avantageusement administrée sous forme d'un produit pharmaceutique, de complément diététique ou alimentaire, de supplément de santé, ou d'un aliment médical/diététique.

La composition se présente sous forme d'un comprimé, une gélule, un gel mou, un semi-solide, un solide, un liquide ou une poudre.

Par ailleurs, les extraits cytoplasmiques de pollens purifiés, spécifiques, standardisés, peuvent être fournis sous la forme de granulés, de poudre ou d'une forme cristalline, en tant que composant des compositions selon la présente invention. Ceci peut être réalisé par lyophilisation, séchage par atomisation, séchage sur cylindre, séchage sous vide des extraits obtenus. De préférence, ledit composant est un extrait séché par atomisation comme décrit au-dessus ou d'un mélange de différents extraits séchés par atomisation comme décrit.

Selon une variante préférée supplémentaire de l'invention, la composition selon la présente invention peut comprendre des excipients choisis parmi le groupe des additifs, tels que les agents glissants, les liants, les agents de délitement, les arômes, les adoucissants, les lubrifiants, les anti-adhérents, les sorbants, les agents de revêtement.

Dans le but de fournir un comprimé avec des aspects améliorant fortement la santé, lesdits un ou plusieurs extraits représentent au moins 40% de la composition totale. Alternativement ou selon une autre variante, lesdits un ou plusieurs extraits sont présents en une quantité de 160 mg à 640 mg de la composition totale. Cette quantité fournit une composition chargée en composés dérivés de pollen et/ou de pistil améliorant la santé.

Les compositions selon la présente invention ont été montrées comme particulièrement utiles pour le traitement de symptômes relatifs aux influences hormonales et à la ménopause chez la femme. Plus particulièrement, lesdites compositions sont utiles pour la femme aussi bien durant la périménopause ou pré-ménopause (la période précédant la ménopause), la ménopause elle-même que la période après la ménopause. Les compositions sont montrées comme permettant de soulager des symptômes et inconforts relatifs.

Le terme « traitement » désigne une amélioration, une prophylaxie ou un renversement d'une maladie ou d'un trouble, ou d'au moins un symptôme discernable de celui-ci. Il s'agit également d'une amélioration, d'une prophylaxie ou d'un renversement d'au moins un paramètre physique mesurable lié à la maladie ou au trouble traité, ce qui n'est pas nécessairement perceptible par le sujet. Dans un autre mode de réalisation, le terme « traitement » désigne l'inhibition ou le ralentissement de la progression d'une maladie ou d'un trouble, soit physiquement, par exemple, la stabilisation d'un symptôme discernable, physiologiquement, par exemple, la stabilisation d'un paramètre physique, ou les deux. Le terme « traitement » désigne également le retard de l'apparition d'une maladie ou d'un trouble. Dans certains modes de réalisation particuliers de l'invention, la composition d'intérêt est administrée en tant que mesure préventive. Dans ce contexte, le terme « prévention » désigne une réduction du risque d'acquisition d'une maladie ou d'un trouble spécifié.

Du fait que la qualité de la composition soit garantie entre les différents lots, les compositions sont montrées comme ayant une efficacité supérieure en comparaison avec les compositions connues de l'art.

Ainsi, l'invention a également pour objet une composition ou un extrait tels que décrits ci-dessus, pour son utilisation dans le traitement des symptômes de la péri-ménopause, post-ménopause et/ou de la ménopause, de préférence la ménopause.

Par traitement des symptômes de la ménopause, on entend le traitement de symptômes tels que les bouffées de chaleur, la transpiration, les palpitations, les troubles cardiaques (battements de coeur inhabituels, arrêts de battement, pincements au coeur), les douleurs musculaires, les maux de tête, l'incontinence à l'effort ou pollakiurie, la dysphorie, les problèmes d'ordre sexuel (changements du désir sexuel, de l'activité sexuel et de la satisfaction), les problèmes vaginaux (sensation de sèchement ou de brûlure dans le vagin, difficulté des rapports sexuels), les douleurs aux articulations, les sautes d'humeur et les mauvaises humeurs, l'humeur dépressive générale, l'irritabilité, l'anxiété, l'affaiblissement physique et mental (diminution générale des performances, mémoire défectueuse, diminution de la concentration, perte de mémoire), problèmes de vessie (difficulté à uriner, augmentation du besoin d'uriner, incontinence de la vessie), inconforts articulaires et musculaires (douleurs dans les articulations, plaintes rhumatoïdes), oedèmes, perte d'énergie, problèmes de sommeil et insomnie (difficulté de s'endormir, à dormir, sommeil léger, réveil anticipé) changement de poids, hypersensibilité.

Par traitement des symptômes de la péri-ménopause (ou pré-ménopause), on entend le traitement de symptômes tels que les tensions nerveuses, la tristesse, l'irritabilité et les angoisses, les douleurs au niveau des seins, les bouffées de chaleur, la migraine, l'augmentation du désir sexuel, la prise de poids, la fatigue, les sueurs froides, les troubles du sommeil ou les douleurs articulaires. La péri-ménopause est une période de transition précédant la ménopause, pendant laquelle la sécrétion des hormones sexuelles se modifie. Ce phénomène est lié à l'épuisement des follicules ovariens associé à une diminution progressive de la sécrétion de progestérone et des oestrogènes. Elle dure quelques années et débute chez des femmes âgées en moyenne de 40 à 45 ans.

Par traitement des symptômes de la post-ménopause, on entend le traitement de symptômes tels que les éblouissements, les sécheresse et démangeaisons vaginales, la prise de poids, l'incontinence liée au stress, la perte osseuse, les infections des voies urinaires, l'insomnie et les bouffées de chaleur occasionnelles. La post-ménopause est définie comme la période qui suit la ménopause. Une femme est considérée en post-ménopause quand elle n'a plus eu de règles pendant une année entière.

De façon avantageuse, comme cela a été montré par le Demandeur, la composition peut être utilisée pour le traitement des bouffées de chaleurs, des troubles du sommeil, des douleurs articulaires, de la modification de l'humeur (sautes d'humeur et les mauvaises humeurs, l'humeur dépressive générale, irritabilité), de la fatigue, et/ou de la modification de la libido. De préférence encore, la composition est utilisée pour le traitement des bouffées de chaleurs.

Le Demandeur a également pu mettre en évidence que la présente composition était efficace lorsqu'elle est utilisée pour le traitement des bouffées de chaleurs induites par l'hormonothérapie chez les patients présentant un cancer, plus particulièrement un cancer de la prostate ou un cancer du sein.

De façon surprenante, le Demandeur a également pu mettre en évidence que la présente composition était efficace chez l'homme. Ainsi, selon un autre aspect, la présente invention a également pour objet une composition ou un extrait tels que décrits ci-dessus, pour son utilisation comme traitement de problèmes d'andropause et d'inconforts relatifs aux déséquilibres hormonaux associés chez l'homme. Le terme andropause est compris comme incluant tous les aspects relatifs aux changements de la balance hormonale chez l'homme, généralement dus à une baisse graduelle de la testostérone. Plus en détails, la composition selon la présente invention peut être utilisée pour le traitement des déséquilibres hormonaux ou des symptômes de l'andropause tels que les bouffées de chaleur, transpirations nocturnes, les troubles cardiaques (conscience inhabituelle des battements, arrêts de battement, emballement du coeur, serrement de la poitrine), les troubles du sommeil (difficulté de s'endormir, à dormir, sommeil léger, réveil anticipé), humeur dépressive (sensation de vague, baisse des activités professionnelles, baisse les activités de loisir, baisse des activités sociales), anxiété (agitation intérieure, attaques de panique), fatigue physique et mentale (diminution générale des performances, mémoire défectueuse, diminution de la concentration, perte de mémoire), problèmes d'ordre sexuel (changement dans le désir sexuel, l'activité sexuel et la satisfaction), problèmes de vessie (difficulté à uriner, augmentation du besoin d'uriner, incontinence de la vessie), inconforts articulaires et musculaires (douleurs dans les articulations, plaintes rhumatoïdes), qualité de vie, changement de poids.

L'invention est ensuite décrite par les exemples suivant non limitatifs qui illustrent l'invention, et qui ne sont pas compris, ni ne peuvent être interprétés, comme limitant la portée de l'invention.

### Exemple 1 : Compositions orales utilisées selon la présente invention

### Composition A

La composition A est un comprimé qui comprend un extrait séché par atomisation obtenu à partir d'un mélange de pollen de maïs (*Zea mays L*.), de seigle (*Secale céréale L.)* et de pin (*Pinus sylvestris L*.) qui est à une concentration de 60% w/w du comprimé. La présence d'oxydase de réticuline et de la protéine de transfert lipidique non spécifique a été confirmée via CPL-SM/SM.

### Composition B

La composition B est un comprimé d'approximativement 380 mg et qui comprend un extrait séché par atomisation obtenu à partir d'un mélange de pollens de maïs (*Zea mays L.*), de seigle (*Secale céréale L.*), de pin (*Pinus sylvestris L*.) et de dactyle (*Dactylis glomerata L*.) et de pistils de maïs (*Zea mays L*.) présents en une quantité en poids dans le comprimé de 160 mg. D'autres ingrédients dans le produit finalisé sont parexemple, la cellulose microcristalline, le stéarate de magnésium et des agents de revêtement. Le talc et la gomme-laque ont été utilisés en tant qu'agent de revêtement. La dose quotidienne recommandée est de deux comprimés par jour, à prendre le matin ou le soir. La présence de l'Oxydase de réticuline, de l'endochitinase A, de la bêta-1,3-glucanase, de l'exopolygalacturonase, et de la protéine de transfert lipidique non spécifique a été confirmée par CPL-SM/SM.

L'ensemble des extraits sont à une concentration comprise entre 35% et 70% w/w du comprimé (composition B).

### Composition C

La composition C est une gélule contenant une poudre, ladite poudre comprenant un premier extrait lyophilisé obtenu à partir d'un mélange de pollen de maïs (*Zea mays L.*), de pistil de maïs (*Zea mays L.*), de pollen de seigle (*Secale cereale L.),* de pollen de dactyle (*Dactylis glomerata L.*) et de pollen de pin (*Pinus sylvestris L.)* ; et un second extrait lyophilisé comprenant du pollen de maïs (*Zea mays L.*), du pollen de seigle (*Secale cereale L)*et du pollen de pin (*Pinus sylvestris L*.).La présence de l'oxydase de réticuline, de l'endochitinase A, de la bêta-1,3-glucanase, de l'exopolygalacturonase, et de la protéine de transfert lipidique non spécifique a été confirmée par CPL-SM/SM.

### Exemple 2 : Contrôle qualité par analyse CPL-SM/SM d'une composition utilisée selon la présente invention

Dans le présent exemple, une composition selon la présente composition C ainsi que l'extrait amenant à la composition finale ont été soumis à un contrôle qualité selon la présente invention. La composition (du produit finalisé) comprend un extrait séché par atomisation obtenu à partir d'un mélange de pollens de maïs (*Zea mays L.*)*,* de seigle (*Secale cereale L*.), de pin (*Pinus sylvestris L*.) et de dactyle (*Dactylis glomerata L*.) et de pistils de maïs (*Zea mays L*.) et a été sujet à une extraction par solvant. Le liquide obtenu a été séparé par électrophorèse sur gel et les bandes de protéines ont été hydrolysées par trypsinisation.

Les extraits utilisés pour la composition ont également été soumis à une électrophorèse sur gel et à une trypsinisation.

Dans une étape ultérieure, les échantillons à la fois de l'extrait et de la composition finale ont été soumis à une procédure CPL-SM/SM et les données obtenues ont été analysées pour détecter la présence d'une des protéines oxydase de réticuline, endochitinase A, bêta-1,3-glucanase, exopolygalacturonase, ou la protéine de transfert lipidique non spécifique.

La présence de l'Oxydase de réticuline, et de la protéine de transfert lipidique non spécifique provenant du pin a été identifiée à la fois dans l'extrait et dans la composition finale, ce qui indique que la composition finale est d'une qualité acceptable et qu'elle peut être commercialisée. Les protéines et/ou les peptides mentionnés comme marqueurs ont été montrés dans des extraits de pollens utilisés pour des études scientifiques pertinentes.

### Exemple 3 : (Bio-) efficacité des compositions utilisées selon la présente invention comparée à des compositions standards

Dans le présent exemple, des compositions qui ont réussi le contrôle qualité (qui est d'avoir au moins une protéine identifiable comme décrit au-dessus) par opposition à des compositions qui ont échoué le contrôle, ont été utilisées dans une démonstration.

Quatre femmes ménopausées (âges : de 51 à 54 ans) avec des symptômes relatifs à la ménopause (bouffées de chaleur, insomnies, sautes d'humeur) ont été divisées au hasard en deux groupes. Le groupe A inclut les patientes qui ont été traitées avec la composition B selon la présente invention, alors que le groupe B inclut les patientes qui ont été traitées avec une composition similaire (d'un point de vue quantitatif) mais de fournisseurs différents (et produite à partir d'une autre méthode d'extraction) et pour laquelle la présence des protéines décrite dans la présente invention n'a pas pu être vérifiée.

Toutes les patientes ont reçu une dose deux fois par jour, pendant trois mois, après lesquels une évaluation a suivi. Les patientes à qui a été donnée la composition selon la présente invention, ont signalé une large diminution des symptômes en comparaison à celles qui ont reçu la composition qui n'était pas selon la présente invention.

### Exemple 4: Utilisation d'une composition selon la présente invention pour le traitement du Syndrome Prémenstruel : deux cas de patientes

Une femme âgée de 32 ans qui n'a pas été enceinte depuis 4 ans souffrait d'un Syndrome Prémenstruel sévère, caractérisé par des bouffées de chaleur, des sautes d'humeur et une irritabilité ou des colères, spécifiquement sept jours avant les menstruations. Les symptômes ont été marqués comme sévères. Aucun autre problème de santé n'a été reporté ou détecté. Le frotti cervico-utérin était normal, ainsi que l'examen gynécologique général.

Dans un premier exemple, des contraceptifs oraux ont été donnés pendant 12 mois, sans aucun succès ou une amélioration des symptômes du Syndrome Prémenstruel. Aucun autre médicament n'a été donné.

Dans une tentative d'éviter l'utilisation d'antidépresseurs, la composition B selon la présente invention, sous forme de comprimé, a été donnée à la patiente (deux fois par jour) pendant trois mois.

Après trois mois, la patiente a été soumise à un questionnaire. Ce dernier montre que la patiente a observé une réduction de tous les symptômes, depuis une condition sévère avant de prendre la composition vers une condition modérée ou légère. Aucun effet secondaire n'a été détecté. Il a été décidé que la patiente devait continuer le traitement.

La seconde patiente est une femme âgée de 38 ans qui souffre d'oedèmes, de privation de sommeil et de sautes d'humeur avant les menstruations. Il lui a été prescrit la composition B selon la présente invention, sous forme de comprimé, (deux fois par jour, pendant quatre mois). Après huit semaines, la patiente a signalé une amélioration de ses symptômes (moins de rétention d'eau, meilleur sommeil et moins de sautes d'humeur). Ces résultats ont été encore meilleurs après quatre mois de traitement. Le traitement a été continué.

### Exemple 5 : Utilisation d'une composition selon la présente invention pour le traitement des symptômes de la ménopause : cas de patientes

Une femme âgée de 57 ans, ménopausée depuis 6 ans avec deux enfants recevait une thérapie de remplacement d'hormone (TRH) depuis 2 ans, dans le but de lutter contre ses symptômes sévères de la ménopause (bouffées de chaleur). La thérapie a été arrêtée de peur d'effets secondaires hormonaux. La patiente prend des médicaments pour une hypertension de stade 2 (enalapril 10 mg par jour) et des médicaments pour la diminution du cholestérol (atorvastatine 20 mg par jour). La patiente souffre également d'un haut taux de glucose et d'un fort IMC, et a été soumise à un régime strict et a reçu des antidiabétiques oraux. Après l'arrêt de la TRH, la patiente a signalé une forte augmentation de ses bouffées de chaleur, au point où celles-ci lui causent des insomnies.

Considérant que la patiente est déjà ménopausée depuis 6 ans, avec un syndrome métabolique et de fortes bouffées de chaleur, il a été décidé de lui donner la composition B selon la présente invention, sous forme de comprimé, deux fois par jour, pendant trois mois.

Après les trois mois d'administration, la patiente a été soumise à un questionnaire pour évaluer les résultats du traitement. La patiente a signé un déclin des symptômes après 5 semaines de prise de la composition. Après trois mois, la quantité de bouffées de chaleur était réduite de moitié, avec une claire diminution de leur intensité. Par conséquent, les problèmes d'insomnies s'étaient dissipés.

Durant un contrôle métabolique, il a été montré que l'administration de la composition n'a eu aucun effet sur aucun des paramètres. Le traitement a dès lors été poursuivi.

La seconde patiente est une femme âgée de 54 ans, souffrant de bouffées de chaleur, de fatigue et de sautes d'humeur causées par une transition ménopausale. Après avoir pris la composition B selon la présente invention, sous forme de comprimé, pendant deux mois, la patiente a signalé moins d'épisodes de bouffées de chaleur, qui étaient également moins prononcés et avec une diminution significative des sautes d'humeur. De plus la fatigue était moins prononcée et son humeur améliorée.

### Exemple 6: Utilisation d'une composition selon la présente invention pour le traitement des bouffées de chaleur chez l'homme : cas d'un patient

Un patient mâle (homme) âgé de 67 ans, traité par une thérapie hormonale pour un cancer de la prostate local et avancé, souffre depuis le début de la thérapie hormonale d'intenses et de fréquentes bouffées de chaleur (6 à 10 par jours), de problèmes de sommeil/d'insomnies et de sautes d'humeur qui ont de manière générale diminué sa qualité de vie (particulièrement au vu des demandes de sa vie professionnelle).

Le patient a reçu la composition B selon la présente invention. Après trois mois d'administration (deux fois par jour), le patient a signalé une forte réduction du nombre de bouffées de chaleur, ainsi que de leur intensité. Ceci a permis de manière générale d'améliorer sa qualité de vie.

### Exemple 7 : Production d'un extrait séché par atomisation selon une variante de la présente invention

La matière végétale requise a été récoltée à la période appropriée et incluait du pollen et des pistils de maïs (*Zea mays L*.), du pollen de seigle (*Secale céréale L.),* de dactyle (*Dactylis glomerata L*.) et de pin (*Pinus sylvestris L.).*

La période appropriée de récolte est :
- juin-juillet pour le pollen de seigle (*Secale céréale L.)* et le pollen de dactyle (*Dactylis glomerata L*.) ;
- juillet-septembre pour le pollen et le pistil de maïs (*Zea mays L*.) ; et
- mai-juin pour le pollen de pin (*Pinus sylvestris L.*)*.*

Ultérieurement, cette matière a été soumise à une étape d'extraction. La matière végétale a été mixée avec 5% d'acétone en milieu aqueux. L'extraction a eu lieu à une température comprise entre 20 et 40°C pendant 24 à 72 heures.

Après extraction, la phase solide et la phase liquide obtenues ont été séparées par centrifugation ou filtration. La phase liquide est gardée de côté.

L'extrait obtenu a été mixé dans une étape ultérieure et séché par atomisation jusqu'à l'obtention d'une poudre.

### Exemple 8 : Caractérisation qualitative et quantitative des marqueurs des pollens et pistils contenus dans les compositions selon l'invention :

### a. Caractérisation qualitative :

La méthode LC-MS est utilisée pour identifier 4 espèces. Les résultats des empreintes peptidiques sont comparés à celles présentes dans la banque de données des protéines végétales des organismes séquencés. Pour l'identification de *Pinus sylvestris*, nous réalisons un test d'immunodiffusion. Les marqueurs ont été choisis après la fabrication de 3 lots industriels. Parmi la liste de protéines identifiées dans l'extrait, nous avons retenu les protéines « traçables » dans les comprimés, repris dans le tableau 1 ci-dessous :

**Tableau 1 : marqueurs ou traceurs associés aux espèces de pollens et/ou pistils :**

| Espèces | Protéines ou peptides identifiés |
|---|---|
| Pollens de Secale cereale L. | Q5TIW8 - allergène de pollen Sec 4 |
| | Q5TIW7 - allergène de pollen Sec 4 |
| | Q1EM97 - Glucanendo-1,3-beta-D-glucosidase |
| Pollens et pistils de Zea Mays L. | B6T5D7 - Reticulase oxidase |
| | E1AFV5 - Beta-13-glucanase |
| | B6UCK8 - Pectinesterase |
| | Q9SPM0- Extensin-like |
| | PGLR2 - Exopolygalacturonase |
| | Q9SYS1 - Beta-amylase |
| | D0EM57 - Chitinase |
| Pollens de Dactylis glomerata L. | Q5TIW3 - allergène de pollen Lol p4 |
| Pollens de Pinus sylvestris L. | Protéine de transfert lipidique, réaction immunologique positive |

### b. Caractérisation quantitative : titre en acides aminés :

A l'extrait natif, sont ajoutés les excipients de nébulisation, maltodextrine, acacia. La composition titre entre 16 g/kg et 38 g/kg en acides aminés totaux par dose journalière.

### Exemple 9 : Production d'un extrait séché par atomisation selon une variante de la présente invention

Le procédé de préparation de l'extrait décrit ici est une extraction aqueuse permettant une sélection maîtrisée des protéines hydrosolubles d'intérêt. La chronologie des opérations, la maîtrise des paramètres de fabrication permet de préserver des protéines ou peptides spécifiques caractéristiques des espèces mises en oeuvre. Ceci assure une traçabilité des espèces dans l'extrait. Le titre de l'extrait est avantageusement standardisé en acides aminés à sa dernière étape de fabrication, lors de la nébulisation. Par cette maîtrise des paramètres de fabrication, une traçabilité, une standardisation du titre en acides aminés et une reproductibilité intra-lots encadrée par une spécification de l'extrait sont assurées.

### i. Préparation des extraits de pollen et de pistils selon le procédé innovant :

La période appropriée de récolte est :
- juin-juillet pour le pollen de seigle (*Secale cereale L.)* et le pollen de dactyle (*Dactylis glomerata L.*) ;
- juillet-septembre pour le pollen et le pistil de maïs (*Zea mays L*.) ;
- mai-juin pour le pollen de pin (*Pinus sylvestris L.*)*.*

Le pollen de pin est récolté à l'état sauvage, les autres pistils et pollen sont issus de cultures agricoles.

Les pollens et pistils sont séchés.

Le procédé peut être caractérisé par les étapes principales suivantes :
a) extraction aqueuse (préférentiellement eau et/ou tensioactif) de pollen ;
b) extraction aqueuse (préférentiellement eau et/ou tensioactif) de pollen et de pistil ;
c) séchage par atomisation des extraits obtenus aux étapes a) et b) ci-dessus ; et
d) récupération desdits extraits de pollen et de pistil de plante(s) obtenus en c).

De façon surprenante, le Demandeur a pu identifier qu'en modifiant les conditions opératoires du procédé de préparation des extraits destinés à l'élaboration des compositions selon l'invention, il était possible d'augmenter la qualité des extraits.

### ii. Détails des étapes principales du procédé préféré selon l'invention :

### a. Etapes d'extraction :

Le Demandeur a pu mettre en évidence que la température des extractions devait être comprise entre 30°C - 45°C.

De préférence, l'extraction est réalisée sous agitation continue, pendant 12 à 90 heures.

### b. Etape de séchage par atomisation du mélange d'extraits :

Les différents extraits, préférentiellement préalablement évaporés, filtrés et/ou décantés, sont mélangés afin d'avoir une substance sèche entre 30% et 50%.

La température de départ de l'étape de séchage par atomisation est comprise entre 138°C et 168°C.

### Exemple 10 : Comparatif des produits issus de deux procédés différents :

Une composition obtenue selon le procédé décrit ci-dessus (exemple 9) est comparée avec une composition obtenue selon le procédé de l'art antérieur, pour lequel la température d'extraction est strictement supérieure à 45°C.

Les deux compositions comprennent les différents pollens et pistils suivants, à des concentrations sensiblement identiques :
- 45%à 90% d'extrait aqueux de pollen de *Secale céréale L.* en poids du poids total de l'extrait ;
- 1% à 35% d'extrait aqueux de pollen de *Zea mays L.* en poids du poids total de l'extrait ;
- 0,01% à 5% d'extrait aqueux de pollen de *Pinus sylvestris L.* en poids du poids total de l'extrait ;
- 3% à 30% d'extrait aqueux de pollen de *Dactylis glomerata L.* en poids du poids total de l'extrait ; et
- 0,1% à 10% d'extrait aqueux de pistil de *Zea mays L.* en poids du poids total de l'extrait.

Le Demandeur a pu constater que la modification de la température d'extraction et des conditions de séparation physiques, ont une incidence sur le résultat des tests d'identification.

Dans les lots d'extraits obtenus selon le procédé de l'art antérieur, on ne retrouve pas la protéine Q5TIW3 (marqueur des pollens de *Dactylis glomerata L*.) alors qu'elle est présente dans les lots d'extraits obtenus selon le procédé décrit à l'exemple 9 ci-dessus.

Il n'y a pas de traçabilité des espèces avec l'ancien procédé.

La mise au point du procédé est une réponse à une exigence de traçabilité et de reproductibilité, tout en intégrant les contraintes économiques industrielles. La recherche des marqueurs/traceurs dans les extraits fabriqués selon les anciennes méthodes et ceux fabriqués avec le procédé selon l'invention montre que les procédés de l'art antérieur ne permettent pas d'assurer la traçabilité des espèces mises en oeuvre.

**Tableau 2 : marqueurs ou traceurs présents dans deux compositions dont les extraits sont obtenus par deux procédés différents :**

| Espèce | Marqueurs des extraits issus du procédé selon l'exemple 9 (T°<45°C) | Marqueurs des extraits issus du procédé selon l'art antérieur (T°C>45°) |
|---|---|---|
| Pollens de Secale cereale L. | Q5TIW8 - allergène de pollen Sec 4 | Q5TIW8 - allergène de pollen Sec 4 |
| Pollens et pistils de Zea Mays L. | B6T5D7 - Reticulase oxidase | B6T5D7 - Reticulase Oxidase |
| | E1AFV5 - Beta-13-glucanase | E1AFV5 non identifié |
| Pollens de Dactylis glomerata L. | Q5TIW3 allergène de pollen Lol p4 | Non identifié |
| Pollens de Pinus sylvestris L. | Protéine de transfert lipidique, réaction immunologique positive | Protéine de transfert lipidique, réaction immunologique positive |

### Exemple 11 : Avantages industriels et qualitatifs additionnels associés au procédé selon l'invention.

De façon avantageuse, le Demandeur réalise l'extraction en une seule opération. Ceci est une simplification ayant des avantages industriels et qualitatifs. Ceci est vrai pour la fabrication de l'extrait et des comprimés.

Ainsi, le nombre d'opérations est divisé par deux, ce qui se traduit par un gain de temps opérationnel et une diminution des risques qualités.

Au niveau de l'extrait : il y a une seule validation, une seule pesée des plantes, une seule extraction, un seul contrôle, un seul nettoyage et un temps d'occupation machine optimisé car les opérations inter-lots sont éliminées. Les risques qualités de contamination croisée ou d'erreur de pesées sont minimisés. Ceci est valable également pour le risque microbiologique qui fait partie de la spécification.

La diminution des risques qualités permet de réduire les « out of spécification » consommateur de temps en investigation et analyse et l'éventuel rejet des lots non conformes.

Ainsi, le procédé objet de l'invention présente notamment les avantages suivants :
- une standardisation améliorée qui garantit un titre constant constitué de la somme des aminoacides, des espèces, résultant de l'extraction ;
- une grande reproductibilité : la validation sur des lots industriels montre que seule la mise au point et la maitrise des paramètres d'extraction permettent d'assurer cette reproductibilité ;
- des bénéfices économiques : par la réduction du nombre d'opérations et l'optimisation de l'utilisation de l'outil industriel.

### Exemple 12 : Utilisation d'une composition obtenue selon le procédé objet de l'invention pour le traitement des bouffées de chaleur liées à la ménopause.

La composition de l'exemple 10 comprenant des extraits obtenus selon le procédé objet de l'invention a été administrée à environ 1360 patientes ménopausées souffrant de bouffées de chaleur.

Le traitement est administré pendant une durée minimum de deux mois aux différentes patientes.

L'amélioration des symptômes chez ces patientes est reprise dans le tableau 3 ci-dessous :

**Tableau 3 : résultat de l'étude multi-centres ménopause :**

| Groupe de patientes | Nombre de patientes en MENOPAUSE vues par semaine | Patientes en MENOPAUSE souffrant des bouffées de chaleur (%) | Taux de satisfaction avec la composition ex. 10 (%) des patientes traitées | Efficacité du traitement avec la composition ex. 10 Echelle de 1 à 5 1=peu efficace 5=très efficace | Durée du traitement avec la composition de l'exemple 10 | Temps pour atteindre l'efficacité du traitement avec la composition de l'exemple 10 |
|---|---|---|---|---|---|---|
| Groupe 1 | 20 | 5 | 100 | 3 | 3 mois | 1 mois |
| Groupe 2 | 20 | 50 | 50 | 3 | en continu | 12 mois |
| Groupe 3 | 10 | 30 | 60 | 3 | 3 mois | 6 mois |
| Groupe 4 | 35 | 70 | 65 | 4 | 2 mois | 2 mois |
| Groupe 5 | 30 | 25 | 75 | 4 | 3 mois | 2 mois |
| Groupe 6 | 15 | 50 | 80 | 4 | en continu | 1,5 mois |
| Groupe 7 | 10 | 50 | 60 | 4 | 6 mois | 2 mois |
| Groupe 8 | 30 | 80 | 70 | 4 | 2 ans | 4 mois |
| Groupe 9 | 20 | 50 | 40 | 4 | 3 mois | 2-3 mois |
| Groupe 10 | 10 | 70 | 60 | 3 | 3 mois | 1,5 mois |
| Groupe 11 | 15 | 70 | 68 | 4 | 3 mois | 3 mois |
| Groupe 12 | 20 | 30 | 30 | 3 | 3 mois | 6 mois |
| Groupe 13 | 20 | 50 | 70 | 4 | 6 mois | 6 mois |
| Groupe 14 | 10 | 70 | 100 | 3 | 3 mois | 4 mois |
| Groupe 15 | 7 | 50 | 50 | 4 | 6 mois | 3 mois |
| Groupe 16 | 4 | 4 | 50 | 4 | 12 mois | 3 mois |
| Groupe 17 | 4 | 90 | 50 | 3 | 3 mois | 3 mois |
| Groupe 18 | 20 | 10 | 60 | 4 | 2 mois | 6 mois |
| Groupe 19 | 5 | 60 | 80 | 4 | en continu | 6 mois |
| Groupe 20 | 20 | 50 | 50 | 3 | 12 mois | 6-12 mois |
| Groupe 21 | 10 | 50 | 90 | 4,5 | 3 mois | 2-3 mois |
| Groupe 22 | 15 | 20 | 40 | 3 | 3 mois | 2-3 mois |
| Groupe 23 | 20 | 80 | 65 | 4 | 3 mois | 3 mois |
| Groupe 24 | 20 | 5 | 50 | 2,5 | 3 | 3 mois |
| Groupe 25 | 25 | 70 | 85 | 4 | 3 | 1,5 mois |
| Groupe 26 | 40 | 85 | 75 | 5 | en continu | 2 mois |
| Groupe 27 | 10 | 90 | 95 | 5 | 3 mois | 1 mois |

Le tableau ci-dessus montre que les patientes ont trouvé le traitement des bouffées de chaleur associée à la ménopause particulièrement efficace.

L'efficacité se fait ressentir très rapidement. Elle se manifeste principalement par la réduction à la fois du nombre, de la fréquence et de la durée des bouffées de chaleurs.

Par ailleurs, certaines patientes ont témoigné spontanément d'amélioration d'autres symptômes liés à la ménopause, tels que :
- les troubles de l'humeur ou l'irritabilité ;
- les troubles du sommeil, de l'insomnie ou de la fatigue ;
- les douleurs articulaires ; et
- les troubles de la libido.

Les médecins ont par ailleurs témoigné que la tolérance au traitement était excellente et qu'ils n'avaient observé aucun effet secondaire

### Exemple 13 : Utilisation d'une composition obtenue selon le procédé objet de l'invention pour le traitement des bouffées de chaleur induites par l'hormonothérapie chez des patients présentant un cancer de la prostate.

La composition de l'exemple 10 comprenant des extraits obtenus selon le procédé objet de l'invention a été administrée à environ 130 patients (hommes) atteints d'un cancer de la prostate souffrant de bouffées de chaleur induites par hormonothérapie.

Le traitement est administré pendant une durée minimum de deux mois aux différents patients.

L'amélioration des symptômes chez ces patientes est reprise dans le tableau 4 ci-dessous :

**Tableau 4 : résultat de l'étude multi-centres hormonothérapie liée au cancer de la prostate :**

| Groupe de patients | Nombre de patients avec un cancer de la prostate | Patients souffrant des bouffées de chaleur (%) | Bouffées de chaleur (BDC) induites par l'hormonothérapie | Taux de satisfaction sur BDC avec la composition de l'exemple 10 (%) des patients traités | Temps pour atteindre une efficacité sur BDC du traitement avec la composition de l'exemple 10 |
|---|---|---|---|---|---|
| Groupe 1 | 7 | 40 | OUI | 50% | non communiqué |
| Groupe 3 | 6 | 50 | OUI | 40% | 6 mois |
| Groupe 4 | 12 | 60 | OUI | 100% | 2 à 3 mois |
| Groupe 5 | 40 | 15 | OUI | 50% | 4 semaines |
| Groupe 6 | 4 | 60 | OUI | 50% | 8 semaines |
| Groupe 7 | 10 | 50 | OUI | 50% | non communiqué |
| Groupe 8 | 10 | 50 | OUI | 50% | 8 semaines |
| Groupe 9 | 6 | 50 | OUI | 40% | 6 mois |
| Groupe 10 | 7 | 40 | OUI | 50% | non communiqué |
| Groupe 11 | 50 | 10 | OUI | 40% | 3 semaines |
| Groupe 12 | 10 | 50 | OUI | 50% | 6 semaines |
| Groupe 13 | 20 | 20 | OUI | 10% | 6 semaines |
| Groupe 14 | 20 | 80 | OUI | 80% | 4 semaines |
| Groupe 15 | 10 | 50 | OUI | 20% | 6 semaines |
| Groupe 16 | 10 | 40 | OUI | 30% | 6 semaines |
| Groupe 17 | 20 | 50 | OUI | 30% | 6 semaines |
| Groupe 18 | 15 | 60 | OUI | 20% | 12 semaines |
| Groupe 19 | 10 | 50 | OUI | 20% | 13 semaines |
| Groupe 20 | 10 | 10 | OUI | 20% | 14 semaines |
| Groupe 25 | 4 | 75 | OUI | 50% | 3 semaines |
| Groupe 26 | 20 | 30 | OUI | 50% | 4 semaines |
| Groupe 27 | 10 | 10 | OUI | 50% | 4 semaines |

Le tableau ci-dessus montre que les patients ont trouvé le traitement des bouffées de chaleur induites par une hormonothérapie particulièrement efficace.

Par ailleurs, l'efficacité se fait ressentir très rapidement. Elle se manifeste principalement par la réduction du nombre, de l'intensité et de la durée des bouffées de chaleurs.

La qualité de vie est ainsi améliorée chez ces patients.

## Revendications

1. Composition orale comprenant :
- un extrait de pollen de seigle (*Secale céréale L.)* ;
- un extrait de pollen de maïs (*Zea mays L*.) ;
- un extrait de pollen de pin (*Pinus sylvestris L*.) ;
- un extrait de pollen de dactyle (*Dactylis glomerata L.*) ; et
- un extrait de pistil de maïs (*Zea mays L*.),
**caractérisée en ce que** lesdits extraits comprennent une protéine ou un peptide dérivé de ladite protéine qui est de la bêta-1,3-glucanase, ladite composition étant préférentiellement sous la forme d'un comprimé, une gélule, un gel mou, un semi-solide, un solide, un liquide ou une poudre.

2. Composition selon la revendication 1, **caractérisée en ce que** lesdits extraits comprennent en outre au moins une deuxième protéine ou un peptide dérivé de ladite deuxième protéine choisis parmi le groupe de l'allergène de pollen Lol p 4, de l'allergène de pollen Sec 4 (*Secale cereale*), la glucanendo-1,3-beta-D-glucosidase, la beta-amylase, la chitinase ou toute combinaison de ces dernières.

3. Composition selon l'une des revendications 1 ou 2, **caractérisée en ce qu'**elle comprend d'une part :
- un extrait de pollen de Secale cereale L. ; 5
- un extrait de pollen de Zea mays L. ;
- un extrait de pollen de Pinus sylvestris L. ;
- un extrait de pollen de Dactylis glomerata L. ; et
- un extrait de pistil de Zea mays L ;
et d'autre part au moins les marqueurs suivants :
- bêta-1,3-glucanase ; et
- l'allergène de pollen Sec 4.

4. Composition selon l'une des revendications précédentes, **caractérisée en ce que** ledit mélange de différents extraits est séché par atomisation.

5. Composition selon l'une quelconques des revendications précédentes, **caractérisée en ce que** les extraits de pollens sont des extraits cytoplasmiques de pollen.

6. Composition selon l'une quelconque des revendications précédentes, **caractérisée en ce qu'**elle est administrée sous forme d'un produit pharmaceutique, de complément diététique ou alimentaire, de supplément de santé, ou d'un aliment médical/diététique.

7. Procédé de préparation d'un extrait aqueux de pollen et de pistil de plante(s) appartenant préférentiellement à la famille des pinacées et/ou des graminées (poacées) comprenant les étapes successives de :
a) extraction aqueuse de pollen ;
b) extraction aqueuse de pollen et de pistil ;
c) séchage par atomisation des extraits obtenus aux étapes a) et b) ;
d) récupération desdits extraits de pollen et de pistil de plante(s) obtenus en c) ;
**caractérisé en ce que** la température des extractions est strictement inférieure à 45°C.

8. Procédé selon la revendication 7, **caractérisé en ce que** la température de départ de l'étape de séchage par atomisation est comprise entre 138°C et 168°C.

9. Procédé selon l'une des revendication 7 ou 8, **caractérisé en ce que** les extractions aqueuses de pollen sont des extractions aqueuses de cytoplasme de pollen

10. Extrait aqueux de pollen, ou de cytoplasme de pollen, et de pistil susceptible d'être obtenu selon le procédé selon l'une des revendications 7 à 9.

11. Composition selon l'une quelconque des revendications 1 à 6 ou extrait selon la revendication 10, pour son utilisation dans le traitement des symptômes de la péri-ménopause, post-ménopause et/ou de la ménopause, de préférence la ménopause.

12. Composition ou extrait pour son utilisation selon la revendication 11, **caractérisée en ce que** lesdits symptômes sont des bouffées de chaleur, des troubles du sommeil, des douleurs articulaires, une modification de l'humeur (sautes d'humeur et les mauvaises humeurs, l'humeur dépressive générale, irritabilité, les tensions nerveuses), de la fatigue, une modification de la libido, des douleurs au niveau des seins, de la migraine, une prise de poids, des sueurs froides, et/ou des douleurs articulaires.

13. Composition selon l'une quelconque des revendications 1 à 6 ou extrait selon la revendication 10, pour son utilisation dans le traitement du syndrome prémenstruel (SPM), préférentiellement dans le traitement des troubles dysphoriques prémenstruels et permettant un apport de tonus et de vitalité, une réduction de la fatigue, d'aider à maintenir une humeur positive, de favoriser le confort avant et pendant les règles.

14. Composition selon l'une quelconque des revendications 1 à 6 ou extrait selon la revendication 10, pour son utilisation dans le traitement des bouffées de chaleurs induites par hormonothérapie chez les patients présentant un cancer, préférentiellement un cancer du sein ou un cancer de la prostate.

15. Composition selon l'une quelconque des revendication 1 à 6 ou extrait selon la revendication 10, pour son utilisation dans le traitement des symptômes de l'andropause et d'inconforts relatifs aux déséquilibres hormonaux associés chez l'homme.

## Patentansprüche

1. Zusammensetzung zur oralen Einnahme umfassend:
- Extrakt aus Roggenpollen (*Secale cererale L*.);
- Extrakt aus Maispollen (*Zea mays L*.);
- Extrakt aus Kiefernpollen (*Pinus sylvestris L*.);
- Extrakt aus Knäuelgraspollen (*Dactylis glomerata L*.);
- Extrakt aus Maisstempeln (*Zea mays L*.);
**dadurch gekennzeichnet, dass** die Extrakte ein Protein oder ein aus dem Protein, das Beta-1,3-Glucanase ist, abgeleitetes Peptid umfassen, wobei die Zusammensetzung vorzugsweise in Form einer Tablette, einer Kapsel, eines weichen Gels, eines Halbfeststoffs, eines Feststoffs, einer Flüssigkeit oder eines Pulvers vorliegt.

2. Zusammensetzung nach Anspruch 1, **dadurch gekennzeichnet, dass** die Extrakte ferner mindestens ein zweites Protein oder ein von dem zweiten Protein abgeleitetes Peptid umfassen, ausgewählt aus der Gruppe Pollenallergen Lol p 4, Pollenallergen Sec 4 (*Secale cereale*), Glucanendo-1,3-beta-D-Glucosidase, beta-Amylase, Chitinase oder einer beliebigen Kombination der letztgenannten.

3. Zusammensetzung nach Anspruch 1 oder 2, **dadurch gekennzeichnet, dass** sie einerseits umfasst:
- Extrakt aus Secale cererale L. Pollen;
- Extrakt aus Zea mays L. Pollen;
- Extrakt aus Pinus sylvestris L. Pollen;
- Extrakt aus Dactylis glomerata L. Pollen; und
- Extrakt aus Zea mays L. Stempel;
und andrerseits mindestens die folgenden Marker:
- Beta-1,3-Glucanase; und
- Pollenallergen Sec 4.

4. Zusammensetzung nach einem der vorhergehenden Ansprüche, **dadurch gekennzeichnet, dass** das Gemisch der verschiedenen Extrakte durch Atomisierung getrocknet wird.

5. Zusammensetzung nach einem der vorhergehenden Ansprüche, **dadurch gekennzeichnet, dass** die Pollenextrakte zytoplasmatische Pollenextrakte sind.

6. Zusammensetzung nach einem der vorhergehenden Ansprüche, **dadurch gekennzeichnet, dass** sie in Form eines pharmazeutischen Produkts als diätetisches oder Nahrungsergänzungsmittel, gesundheitsfördernden Ergänzungsmittel oder medizinisch/diätetisches Lebensmittel verabreicht wird.

7. Verfahren zur Herstellung eines wässrigen Extrakts aus Pollen und Stempeln von Pflanzen, die vorzugsweise zur Familie der Pinaceae und/oder Gräser (Poaceae) gehören, aufeinanderfolgende Schritte umfassend:
a) wässrige Extraktion von Pollen;
b) wässrige Extraktion von Pollen und Stempel;
c) Trocknen durch Atomisierung der aus den Schritten a) und b) gewonnenen Extrakte;
d) Rückgewinnung der in c) erhaltenen Pollen- und Stempelextrakte der Pflanze(n);
**dadurch gekennzeichnet, dass** die Extraktionstemperatur strikt unter 45°C liegt.

8. Verfahren nach Anspruch 7, **dadurch gekennzeichnet, dass** die Temperatur ab dem Schritt der Trocknung durch Atomisierung zwischen 138°C und 168°C liegt.

9. Verfahren nach Anspruch 7 oder 8, **dadurch gekennzeichnet, dass** wässrige Extraktionen von Pollen wässrige Extraktionen von Pollenzytoplasma sind.

10. Wässriger Extrakt aus Pollen oder Pollenzytoplasma und Stempel, erhältlich nach dem Verfahren nach einem der Ansprüche 7 bis 9.

11. Zusammensetzung nach einem der Ansprüche 1 bis 6 oder Extrakt nach Anspruch 10, zur Verwendung bei der Behandlung von Symptomen der Perimenopause, Postmenopause und/oder Menopause, vorzugsweise der Menopause.

12. Zusammensetzung oder Extrakt nach Anspruch 11, **dadurch gekennzeichnet, dass** die Symptome Hitzewallungen, Schlafstörungen, Gelenkschmerzen, Veränderung der Stimmungslage (Stimmungsschwankungen und schlechte Laune, allgemeine depressive Verstimmung, Reizbarkeit, nervöse Anspannung), Müdigkeit, Veränderung der Libido, Brustschmerzen, Migräne, Gewichtszunahme, kalter Schweiß und/oder Gelenkschmerzen sind.

13. Zusammensetzung nach einem der Ansprüche 1 bis 6, oder Extrakt nach Anspruch 10 zur Verwendung bei der Behandlung des prämenstruellen Syndroms (PMS), vorzugsweise bei der Behandlung von prämenstruellen dysphorischen Störungen und eine Zufuhr von Tonus und Vitalität, eine Verringerung der Müdigkeit ermöglichend, wobei zur Aufrechterhaltung einer positiven Stimmung beigetragen wird und das Wohlbefinden während der Menstruation gefördert wird.

14. Zusammensetzung nach einem der Ansprüche 1 bis 6, oder Extrakt nach Anspruch 10 zur Verwendung bei der Behandlung von durch Hormontherapie induzierten Hitzewallungen bei Patienten mit Krebs, vorzugsweise Brustkrebs oder Prostatakrebs.

15. Zusammensetzung nach einem der Ansprüche 1 bis 6, oder Extrakt nach Anspruch 10 zur Verwendung bei der Behandlung von Symptomen der Andropause sowie Beschwerden im Zusammenhang der damit verbundenen hormonellen Ungleichgewichte bei Männern.

## Claims

1. Oral composition comprising:
- an extract of rye pollen (*Secale cereale L.*);
- an extract of maize pollen (*Zea mays L*.);
- an extract of pine pollen *(Pinus sylvestris L.)* ;
- an extract of orchard grass pollen *(Dactylis glomerata L.)*; and
- an extract of maize pistil (*Zea mays L.),*
**characterised in that** said extracts comprise a protein or peptide derived from said protein which is beta-1,3-glucanase, said composition being preferably in the form of a tablet, capsule, soft gel, semi-solid, solid, liquid or powder.

2. Composition according to claim 1, **characterised in that** said extracts further comprise at least one second protein or peptide derived from said second protein selected from the group of pollen allergen Lol p 4, pollen allergen Sec 4 (*Secale cereale*), glucanendo-1,3-beta-D-glucosidase, beta-amylase, chitinase or any combination thereof.

3. Composition according to one of claims 1 or 2, **characterised in that**, on one hand, it involves:
- an extract of Secale cereale L. pollen;
- an extract of Zea mays L. pollen;
- an extract of Pinus sylvestris L. pollen;
- an extract of Dactylis glomerata L pollen; and
- an extract of Zea mays L. pistil;
and on the other hand, at least the following markers:
- beta-1,3-glucanase; and
- pollen allergen Sec 4.

4. Composition according to one of the preceding claims, **characterised in that** said mixture of different extracts is spray-dried.

5. Composition according to any of the preceding claims, **characterised in that** the pollen extracts are cytoplasmic pollen extracts.

6. Composition according to any one of the preceding claims, **characterised in that** it is administered as a pharmaceutical, dietary or food supplement, health supplement, or medical/dietary food product.

7. Process for preparing an aqueous extract of pollen and pistil, obtained from plant(s) preferably belonging to the Pinaceae and/or grass family (Poaceae) that comprises the successive stages of:
a) aqueous extraction of pollen;
b) aqueous extraction of pollen and pistil;
c) spray drying the extracts obtained in steps a) and b);
d) recovery of said extracts of pollen and pistil from plant(s) obtained in c);
**characterised in that** the temperature of the extractions is strictly below 45°C.

8. Process according to claim 7, **characterised in that** the starting temperature of the spray drying step is between 138°C and 168°C.

9. Process according to any of claims 7 or 8, **characterised in that** the aqueous pollen extractions are aqueous pollen cytoplasm extractions.

10. Aqueous extract of pollen, or pollen cytoplasm, and pistil likely to be obtained by the process according to one of claims 7 to 9.

11. Composition according to any of the claims from 1 to 6 or extract according to claim 10 for its use in the treatment of the symptoms of perimenopause, post menopause and/or menopause, preferably menopause.

12. Composition or extract for its use according to claim 11, **characterised in** which the said symptoms are hot flushes, trouble with sleeping, joint pains, mood changes (mood swings and bad moods, general depressive mood, irritability, nervous tensions), fatigue, change in libido, breast pains, migraine, weight gain, cold sweats and/or joint pains.

13. Composition according to any of the claims from 1 to 6 or extract according to claim 10 for its use in the treatment of premenstrual syndrome (PMS), preferably in the treatment of premenstrual dysphoric problems and enabling a supply of tonicity and vitality, a reduction in fatigue, helping to maintain a positive mood, and favourising comfort before and during the menstruations.

14. Composition according to any of the claims from 1 to 6 or extract according to claim 10 for its use in the treatment of hot flushes induced by hormonotherapy in patients with cancer, preferably breast cancer or prostate cancer.

15. Composition according to any of the claims from 1 to 6 or extract according to claim 10 for its use in the treatment of the symptoms of andropause and discomfort related to hormonal imbalances in men.
